# FASCICULE DE BREVET EUROPEEN

(11) **EP 3 675 960 B1**
(45) Date de publication et mention de la délivrance du brevet: **27.03.2024**
(21) Numéro de dépôt: 18758631.8
(22) Date de dépôt: 27.08.2018
(51) Int. Cl.: A61K 36/15, A61K 36/899, A61K 31/355, A61P 15/08, A61K 31/122, A61K 45/06

(54) **COMPOSITION POUR SON UTILISATION DANS LE TRAITEMENT ET/OU LA PREVENTION DE L'INFERTILITE**
ZUSAMMENSETZUNG ZUR VERWENDUNG BEI DER BEHANDLUNG UND / ODER PRÄVENTION VON UNFRUCHTBARKEIT
COMPOSITION FOR USE OF SAME IN THE TREATMENT AND/OR PREVENTION OF INFERTILITY

(30) Priorité: 28.08.2017 FR 1770894
(43) Date de publication de la demande: 08.07.2020
(73) Titulaire: AXEEN PHARMA S.A.R.L., 98000 Monaco (MC)
(72) Inventeur: MORRA, Sossio, 98000 Monaco (MC)
(74) Mandataire: Axe PI
(86) Numéro de dépôt international: PCT/EP2018/073018
(87) Numéro de publication internationale: WO 2019/042934

(56) Documents cités:
- EP-A1- 2 135 621
- WO-A1-02/17944
- FR-A1- 2 964 834
- Mark A Moyad: "Review of Lifestyle and CAM for Miscellaneous Urologic Topics" In: "Complementary & Alternative Medicine for Prostate and Urologic Health", 1 janvier 2014 (2014-01-01), Springer, XP055450356, page FP1-2, 236-238, page 237 - page 238
- Anonymous: "Graminex Flower Pollen Extract and its Effect on Fertility", , 25 octobre 2009 (2009-10-25), pages 1-9, XP055450407, Extrait de l'Internet: URL:http://www.graminex.com/PDFs/clinicals /Clinicals_Effect_on_Fertility.pdf [extrait le 2018-02-12] cité dans la demande
- YONGFANG LEI ET AL: "Protective effects of grape seed-derived procyanidin extract against carrageenan-induced abacterial prostatitis in rats", JOURNAL OF FUNCTIONAL FOODS, vol. 7, 5 février 2014 (2014-02-05), pages 416-424, XP055450419, NL ISSN: 1756-4646, DOI: 10.1016/j.jff.2014.01.016
- ELIST ET AL: "Effects of pollen extract preparation Prostat/Poltit on lower urinary tract symptoms in patients with chronic nonbacterial prostatitis/chronic pelvic pain syndrome: A randomized, double-blind, placebo-controlled study", UROLOGY, BELLE MEAD, NJ, US, vol. 67, no. 1, 1 janvier 2006 (2006-01-01), pages 60-63, XP027928161, ISSN: 0090-4295 [extrait le 2006-01-01] cité dans la demande
- AKRAM M. EL-KASHLAN ET AL: "Therapeutic Potential of Date Palm Pollen for Testicular Dysfunction Induced by Thyroid Disorders in Male Rats", PLOS ONE, vol. 10, no. 10, 1 octobre 2015 (2015-10-01), page e0139493, XP055450552, DOI: 10.1371/journal.pone.0139493
- AFSANEH KHADEMI ET AL: "The effect of L-carnitine on sperm parameters in patients candidatedfor Intracytoplasmic Sperm Injection", IRANIAN JOURNAL OF REPRODUCTIVE MEDICINE,, vol. 2, no. 2, 1 janvier 2004 (2004-01-01) , pages 65-69, XP002765319, cité dans la demande
- HARRY G. PREUSS ET AL: "A Critical Review of Cernitin(TM) for Symptomatic Relief Of Lower Urinary Tract Symptoms (LUTS) in Men", RESEARCH COMMUNICATIONS IN PHARMACOLOGY AND TOXICOLOGY, 1 janvier 2013 (2013-01-01), page FP, 7-15, XP055450115,
- ANN-CATHRIN HELLSTR?M ET AL: "The pollen extract Femal-a nonestrogenic alternative to hormone therapy in women with menopausal symptoms", MENOPAUSE, vol. 19, no. 7, 1 juillet 2012 (2012-07-01), pages 825-829, XP055354304, US ISSN: 1072-3714, DOI: 10.1097/gme.0b013e31824017bc
- Taylor: "Alternative approaches to menopause" In: "Postmenopausal Endocrinology", 1 janvier 2015 (2015-01-01), Elsevier, XP055450674, page FP1-3,635,636,646, page 636
- K WINTHER ET AL: "Femal, a herbal remedy made from pollen extracts, reduces hot flushes and improves quality of life in menopausal women: a randomized, placebo-controlled, parallel study", CLIMACTERIC, vol. 8, no. 2, 3 juin 2005 (2005-06-03), pages 162-170, XP055354258, GB ISSN: 1369-7137, DOI: 10.1080/13697130500117987
- PIOTR CZUCZWAR ET AL: "The safety and tolerance of phytotherapies in menopausal medicine - a review of the literature", PRZEGLAD MENOPAUZALNY - MENOPAUSE REVIEW, vol. 1, 26 avril 2017 (2017-04-26), pages 8-11, XP055419027, PL ISSN: 1643-8876, DOI: 10.5114/pm.2017.67365
- MAGGIONI CRISTINA: "CREALYS : New Product for Man Infertility in-vivo and in-vitro Results", ADVANCEMENTS IN CASE STUDIES, vol. 2, no. 4, 14 April 2020 (2020-04-14), XP093102368, DOI: 10.31031/AICS.2020.02.000541 Retrieved from the Internet: URL:https://crimsonpublishers.com/aics/pdf /AICS.000541.pdf>
- Marcello Locatelli ET AL: "Graminex Pollen: Phenolic Pattern, Colorimetric Analysis and Protective Effects in Immortalized Prostate Cells (PC3) and Rat Prostate Challenged with LPS", Molecules, vol. 23, no. 5, 1 May 2018 (2018-05-01), page 1145, XP055762491, DE ISSN: 1433-1373, DOI: 10.3390/molecules23051145

## Description

La présente invention concerne une composition comprenant :
- un extrait aqueux de pollen de Secale céréale L. ;
- un extrait aqueux de pollen de Zea mays L. ;
- un extrait aqueux de pollen de Pinus silvestris L. ;
- un extrait aqueux de pollen de Dactylis glomerata L. et
- un extrait aqueux de pistil de Zea mays L.,
pour son utilisation dans le traitement et/ou la prévention de l'infertilité, chez des hommes présentant une oligospermie, une cryptozoospermie, une azoospermie, une asthénospermie, une oligoasthénospermie et/ou une tératospermie, caractérisé en ce que l'extrait de pollen et l'extrait de pistil sont des extraits aqueux dont l'extraction est réalisée à une température inférieure à 45°C.

L'infertilité peut être considérée comme l'incapacité d'un couple à obtenir une grossesse après environ une année de rapports sexuels réguliers non protégés, ou l'incapacité d'une femme à mener une grossesse jusqu'à terme. On parle aussi de stérilité.

En moyenne, plus d'un couple sur dix est amené à consulter un médecin pour des problèmes d'infertilité. Une étiologie masculine est retrouvée dans près de 40 % des cas.

Une fertilité masculine normale est généralement liée aux conditions suivantes :
- une production normale des spermatozoïdes par les testicules (spermatogénèse), en qualité comme en quantité ;
- une bonne circulation des spermatozoïdes au sein des organes génitaux masculins, ce qui implique toute absence d'obstacle au niveau des épididymes, des canaux déférents et de l'urètre ; et
- une éjaculation adéquate.

Tout facteur pouvant entraver l'un de ces mécanismes peut être responsable d'une hypofertilité, voire d'une infertilité (stérilité), chez l'homme.

L'analyse du sperme est l'examen le plus important dans l'évaluation de la fertilité masculine. Le spermogramme de base, ou spermocytogramme mesure avec précision des paramètres comme notamment le volume du sperme, le pH, et la présence de leucocytes dans le sperme, le nombre des spermatozoïdes, la mobilité des spermatozoïdes dans la première demi-heure, la morphologie des spermatozoïdes ainsi que leur vitalité.

Les résultats du spermogramme peuvent varier en fonction notamment du stress, de la durée de l'abstinence, de l'absorption d'alcool, de médicaments ou de drogues.

Afin de juger de la qualité du sperme, certaines données sont prises en considération à partir de l'éjaculat (normes Organisation Mondiale de la Santé), telles que notamment :
i. le volume du sperme (volume normal compris entre 1,5 ml et 6 ml) :
   - si le volume est égal à 0 ml, on parle d'aspermie ;
   - si le volume est supérieur à 6 ml, on parle d'hyperspermie ;
   - si le volume est inférieur à 1,5 ml, on parle d'hypospermie.
ii. Le pH du sperme qui doit être compris entre 7.2 et 8.
iii. La concentration des spermatozoïdes (concentration normale comprise entre 15 millions et 200 millions par ml de sperme) :
   - si la concentration est supérieure à 200 millions/ml, on parle de polyspermie ;
   - si la concentration est inférieure à 15 millions/ml, on parle d'oligospermie ;
   - si la concentration est inférieure à 5 millions/ml, on parle d'oligospermie sévère ;
   - si la concentration est inférieure à 1 million/ml, on parle plus particulièrement de cryptozoospermie ; et
   - en l'absence totale de spermatozoïdes dans l'éjaculat, on parle d'azoospermie.

Chez les patients présentant un problème de concentration des spermatozoïdes, on distingue généralement les patients présentant une oligospermie de ceux présentant une cryptozoospermie ou une azoospermie.

En effet, l'examen microscopique direct d'une goutte de sperme ne permet pas d'observer la présence de spermatozoïdes dans le cas d'une cryptozoospermie ou d'une azoospermie, au contraire d'une oligospermie même sévère.

Il est alors nécessaire de réaliser une recherche approfondie par centrifugation du sperme et examen du culot de centrifugation. Lorsqu'elle permet d'en retrouver quelques-uns (moins d'1 million de spermatozoïdes par ml), on parle de cryptozoospermie, l'absence totale de spermatozoïdes dans l'éjaculat révélant une azoospermie.

Par ailleurs, on peut distinguer deux formes distinctes d'azoospermie :
- on parle d'azoospermie « excrétoire » ou « obstructive » lorsque les spermatozoïdes sont correctement produits au niveau des testicules, mais qu'il existe un problème à un niveau quelconque du transport des spermatozoïdes dans le tractus génital masculin (épididymes, canaux déférents, canaux éjaculateurs), de telle sorte que ces spermatozoïdes n'atteignent pas l'éjaculat ; et
- on parle d'azoospermie « sécrétoire » ou « non obstructive » lorsqu'il n'y a pas de production de spermatozoïdes par les testicules.
iv. La mobilité totale des spermatozoïdes peut être classée en :
- progressive : lorsque les spermatozoïdes bougent activement indépendamment de la vitesse ;
- non progressive : lorsque les spermatozoïdes bougent mais sans progression ; et
- immobile : lorsqu'il n'y a pas de mouvement.

Un spermogramme est considéré comme normal si la mobilité totale (progressive et non progressive) est supérieure à 40 %.

Cette mobilité des spermatozoïdes est un autre paramètre à considérer dans la prévention ou le traitement de l'infertilité masculine. En effet, dès qu'une asthénospermie, c'est-à-dire une faible mobilité des spermatozoïdes (plus de 65% sont immobiles) vient s'ajouter à une oligospermie, le pouvoir fécondant devient très faible et tend vers zéro pour des numérations inférieures à 10 millions/ml.
v. La morphologie des spermatozoïdes est également prise en considération :
- si la morphologie est inférieure à 4% (Krueger) de formes normales de spermatozoïdes, on parle de tératospermie.

Aussi, l'infertilité masculine est souvent due à un nombre insuffisant de spermatozoïdes, à un défaut de mobilité ou un taux élevé de spermatozoïdes anormaux, à un défaut du pH, à un volume anormal de sperme, au manque de vitalité de spermatozoïdes et/ou à la présence anormale de leucocytes (concentration normale inférieure à 1 million/ml), ces anomalies pouvant être combinées, par exemple chez des hommes oligoasthénospermiques.

Il y a également d'autres facteurs associés à l'infertilité masculine qui peuvent être le résultat d'anomalies au niveau de la pénétration de spermatozoïdes qui sont liés aux anticorps, et au niveau de concentrations de certains éléments dans le sperme comme le zinc, l'alpha glucosidase et le fructose.

Au niveau moléculaire, l'infertilité peut être associée aux dérèglements de certaines voies de transduction du signal, à une production excessive de dérivés réactifs de l'oxygène (DRO, ou ROS en anglais pour Reactive Oxygen Species) ou encore à une activité excessive d'enzymes comme la créatine phosphokinase. L'infertilité peut aussi se rechercher au niveau de l'ADN.

Les azoospermies d'origine sécrétoire ont pendant de nombreuses années été considérées comme des causes de stérilité définitive nécessitant nécessairement le recours à un don de sperme par le couple, ou à l'adoption.

De même, une quantité insuffisante de spermatozoïdes dans le sperme d'hommes cryptozoospermiques peut conduire à une absence de fertilité naturelle et nécessiter le recours à un don de sperme par le couple, ou à l'adoption.

Néanmoins, avant de se tourner vers le don de sperme ou l'adoption, la plupart des couples concernés ont d'abord recours à de nouvelles techniques de fécondation *in vitro* qui consistent à ponctionner les testicules ou épididymes de manière à y recueillir les spermatozoïdes qui s'y trouvent, telles que l'ICSI (Intra Cytoplasmic Sperm Injection) avec laquelle il suffit en théorie de quelques spermatozoïdes éjaculés ou prélevés dans l'épididyme ou dans le testicule, indépendamment de la concentration et de la mobilité spermatique, pour obtenir une fécondation et une grossesse, ou encore l'IMSI (Intracytoplasmic Morphological Sperm Injection) qui permet de faire une sélection très poussée des meilleurs spermatozoïdes dans l'éjaculat et d'augmenter de façon significative les chances d'obtenir une fécondation et donc un enfant.

Ce pouvoir fécondant n'est toutefois jamais nul, et tout couple peut connaître des grossesses authentiques attribuables à l'homme malgré une oligoasthénospermie sévère.

L'infécondité masculine peut dans quelques cas être traitée en ayant recourt à des techniques chirurgicales ou médicales, mais les résultats demeurent malheureusement souvent décevants. C'est pourquoi les spécialistes se tournent de plus en plus vers les techniques d'assistance médicale à la procréation (AMP) dont les résultats sont parfois plus satisfaisants.

En pratique, il n'est toutefois pas question d'attendre cette éventualité rare, et il est important de tenter d'améliorer la qualité du sperme pour augmenter les chances d'avoir une fécondation naturelle et ainsi éviter d'avoir recours à des techniques de fécondation *in vitro* très coûteuses qui consistent à ponctionner les testicules ou épididymes de manière à y recueillir les spermatozoïdes qui s'y trouvent, telles que l'ICSI ou encore l'IMSI, à un don de sperme, ou encore à l'adoption.

De nombreuses études sur les causes d'infertilité et leurs traitements ont déjà été réalisées.

Il est par exemple connu d'après la publication « Effect of Palm Pollen on Sperm Parameters of Infertile Man » (Athar Rasekh et al., Pakistan Journal of Biological Sciences 18 (4): 196-199, 2015) que la consommation de pollen de palmier (DPP pour Phoenix dactylifera Date Palm Pollen) permet d'améliorer le nombre et la mobilité des spermatozoïdes.

La publication « Effects of pollen extract EA-10, P5 on chronic prostatitis or infertility with chronic prostatitis » (Acta Pharmacol Sin. 2002 Nov ;23(11):1035-9., Chen et al.) décrit également les effets d'extraits huileux et aqueux de pollen EA-10, P5 sur la prostatite chronique et sur l'infertilité liée à la prostatite chronique. Toutefois, cette publication recommande d'associer les extraits de pollen à un antibiotique tel que la roxithromycine en vue d'améliorer la prostatite chronique et l'infertilité liée à la prostatite chronique.

La publication « *Graminex Flower Pollen Extract and its Effect on Fertility* » regroupe différentes publications qui divulguent notamment que l'extrait de pollen EA-10, P5 a été utilisé dans le traitement de l'infertilité associée à une prostatite chronique.

La publication « Effects of pollen extract préparation PROSTAT/POLTIT on lower urinary tract symptoms in patients with chronic nonbacterial prostatitis/chronic pelvic pain syndrome : a randomized, double-blind, placebocontrolled Study », (Urology 67 (1), 2006, James Elist) décrit également que le produit PROSTAT/POLTIT comprenant également des extraits huileux et aqueux de pollen EA-10, P5 (74 mg d'extrait de pollen des espèces *Graminae*) est efficace contre les dysfonctionnement sexuels (libido, érection, problèmes d'éjaculation, etc.)

Le produit PROSTAL^{™} des laboratoires MotiMa contient des extraits purifiés, hydrosolubles et liposolubles, de différents types de pollen tels que les extraits de pollen P5 et de pollen EA-10. PROSTAL^{™} est connu à ce jour comme adjuvant naturel pour faire face aux gênes urinaires et aux baisses de tonus général, facteur de performances sexuelles.

La publication Khademi et al., 2004 divulgue que le traitement avec de la L-carnitine améliore la qualité des spermatozoïdes chez l'homme.

La demande FR2964834 divulgue une composition comprenant des vitamines B6, B9, B12, C, E, du zinc, de la carnitine, de la coenzyme Q10 et du lycopène pour traiter l'infertilité et assurer une protection des spermatozoïdes.

Enfin la demande EP2135621 divulgue une composition pour traiter l'infertilité masculine comprenant de l'arginine, des vitamines A, C, E, B6, B9, B12, de la coenzyme Q10, de la L-carnitine, du zinc et du sélénium.

Toutefois, aucun des documents ci-dessus ne met en évidence ni ne suggère un quelconque effet potentialisé de l'association de plusieurs pollens et/ou pistils principalement obtenus à partir de plantes appartenant à la famille des pinacées et/ou des poacées, sur l'augmentation de la production des spermatozoïdes et/ou de leur mobilité chez des hommes hypofertiles ou infertiles, notamment chez des hommes présentant une oligoasthénospermie, qui constitue un groupe de patients spécifique. Par ailleurs, aucun des documents ci-dessus ne suggère que l'association de plusieurs pollens et/ou pistils principalement obtenus à partir de plantes appartenant à la famille des pinacées et/ou des poacées puisse avoir un effet positif sur les paramètres du spermogramme tels que notamment le volume, pH, nombre, mobilité, concentration, morphologie, vitalité, ou et/ou la présence de leucocytes, etc.

Enfin, aucun des documents ci-dessus ne suggère que l'association de plusieurs pollens et/ou pistils principalement obtenus à partir de plantes appartenant à la famille des pinacées et/ou des poacées puisse avoir un effet positif sur la pénétration de spermatozoïdes ou au niveau de concentrations de certains éléments dans le sperme comme le zinc, l'alpha glucosidase et le fructose ou au niveau moléculaire, impliquant certains voies de transduction du signal, ou impliquant des enzymes comme la créatine phosphokinase ou au niveau de l'ADN.

Considérant ce qui précède, un problème que se propose de résoudre la présente invention est de mettre en oeuvre une composition apte à améliorer la production et/ou la qualité des spermatozoïdes par les testicules chez des hommes, de sorte à améliorer leur fécondité, et ainsi leur éviter de devoir se tourner vers la fécondation *in vitro,* le don de sperme ou l'adoption pour avoir un enfant.

Ainsi, le Demandeur a découvert, de manière inattendue, qu'une composition comprenant un extrait aqueux de pollen de Secale céréale L. ;
- un extrait aqueux de pollen de Zea mays L. ;
- un extrait aqueux de pollen de Pinus silvestris L. ;
- un extrait aqueux de pollen de Dactylis glomerata L. et
- un extrait aqueux de pistil de Zea mays L., caractérisé en ce que l'extrait de pollen et l'extrait de pistil sont des extraits aqueux dont l'extraction est réalisée à une température inférieure à 45°C, permettait d'augmenter la production des spermatozoïdes chez des hommes oligospermiques, leur vitalité et/ou leur mobilité chez des hommes asthénospermiques. Une telle composition prise seule et, avantageusement, en association avec une pluralité d'autres principes actifs agissant en synergie, présente une remarquable activité potentialisée notamment sur la mobilité, la vitalité et la production des spermatozoïdes.

La solution de l'invention à ce problème technique posé a pour premier objet une composition comprenant :
- un extrait aqueux de pollen de *Secale cereale* L. ;
- un extrait aqueux de pollen de Zea *mays* L. ;
- un extrait aqueux de pollen de *Pinus silvestris* L. ;
- un extrait aqueux de pollen de *Dactylis glomerata* L. ; et
- un extrait aqueux de pistil de *Zea mays* L. ;

pour son utilisation dans le traitement et/ou la prévention de l'infertilité, chez des hommes présentant une oligospermie, une cryptozoospermie, une azoospermie, une asthénospermie, une oligoasthénospermie et/ou une tératospermie. Préférentiellement la pathologie à traiter est une asthénospermie et/ou une tératospermie,
caractérisé en ce que l'extrait de pollen et l'extrait de pistil sont des extraits aqueux dont l'extraction est réalisée à une température inférieure à 45°C.

Divulgué mais pas revendiqué est un procédé de préparation d'un extrait aqueux de pollen et de pistil de plante(s) appartenant préférentiellement à la famille des pinacées et/ou des graminées (poacées) comprenant les étapes successives de :
a) extraction aqueuse de pollen ;
b) extraction aqueuse de pollen et de pistil ;
c) séchage par atomisation des extraits obtenus aux étapes a) et b) ;
d) récupération desdits extraits de pollen et de pistil de plante(s) obtenus en c) ;
caractérisé en ce que la température des extractions est strictement inférieure à 45°C.

Divulgué mais pas revendiqué est un extrait aqueux de pollen et de pistil susceptible d'être obtenu selon le procédé divulgué mais pas revendiqué, et son utilisation dans le traitement et/ou la prévention de l'infertilité.

L'invention et les avantages qui en découlent seront mieux compris à la lecture de la description et des modes de réalisation non limitatifs qui suivent.

Dans cette description, à moins qu'il ne soit spécifié autrement, il est entendu que, lorsqu'un intervalle est donné, il inclut les bornes supérieure et inférieure dudit intervalle.

L'invention concerne une composition comprenant :
- un extrait aqueux de pollen de Secale céréale L. ;
- un extrait aqueux de pollen de Zea mays L. ;
- un extrait aqueux de pollen de Pinus silvestris L. ;
- un extrait aqueux de pollen de Dactylis glomerata L. ; et
- un extrait aqueux de pistil de Zea mays L.,
pour son utilisation dans le traitement et/ou la prévention de l'infertilité, chez des hommes présentant une oligospermie, une cryptozoospermie, une azoospermie, une asthénospermie, une oligoasthénospermie et/ou une tératospermie, caractérisé en ce que l'extrait de pollen et l'extrait de pistil sont des extraits aqueux dont l'extraction est réalisée à une température inférieure à 45°C.

Le terme « traitement » désigne une amélioration, une prophylaxie ou un renversement d'une maladie ou d'un trouble, ou d'au moins un symptôme discernable de celui-ci. Il s'agit également d'une amélioration, d'une prophylaxie ou d'un renversement d'au moins un paramètre physique mesurable lié à la maladie ou au trouble traité, ce qui n'est pas nécessairement perceptible par le sujet. Dans un autre mode de réalisation, le terme « traitement » désigne l'inhibition ou le ralentissement de la progression d'une maladie ou d'un trouble, soit physiquement, par exemple, la stabilisation d'un symptôme discernable, physiologiquement, par exemple, la stabilisation d'un paramètre physique, ou les deux. Le terme « traitement » désigne également le retard de l'apparition d'une maladie ou d'un trouble. Dans certains modes de réalisation particuliers de l'invention, la composition d'intérêt est administrée en tant que mesure préventive. Dans ce contexte, le terme « prévention » désigne une réduction du risque d'acquisition d'une maladie ou d'un trouble spécifié.

Selon l'invention, on parle d'extraction lorsqu'on utilise l'eau en tant que solvant sur une matière première végétale pour en extraire certains composés ou molécules, après un éventuel mélange, décantation et filtration.

Le solvant peut être ensuite éliminé partiellement ou totalement pour obtenir un extrait.

Après leur ramassage, les pollens peuvent être utilisés frais ou séchés, avantageusement séchés, et éventuellement débactérisés.

La composition utilisée selon l'invention comprend un extrait aqueux de pollen et un extrait aqueux de pistil.

Par extrait aqueux, on entend un extrait contenant des principes actifs hydrosolubles obtenu par extraction, par exemple une hydrodistillation, une macération, une infusion, une digestion, une décoction, une percolation ou encore une lixiviation, préférentiellement une macération à basse température comprise entre 15 et 40°C, d'une matière première végétale dans un solvant aqueux, c'est-à-dire un solvant comprenant de l'eau prise seule ou avantageusement en mélange avec d'autre(s) solvant(s) tels qu'un alcool, une cétone, et/ou un tensioactif non ionique.

A titre d'exemples non limitatifs, le solvant aqueux est choisi parmi un mélange comprenant majoritairement de l'eau en combinaison avec un alcool tel que l'éthanol, une cétone telle que l'acétone, et/ou tensioactif non ionique.

Les extraits de pollen et de pistil utilisés dans la composition pour son utilisation selon l'invention sont obtenus à partir de plantes selon la revendication 1.

Les poacées (Poaceae), appelée également graminées *(Gramineae),* sont une famille de plantes monocotylédones de l'ordre des poales. Cette famille, composée d'environ 12 000 espèces regroupées en 780 genres, comprend la plupart des espèces appelées communément « herbes » et « céréales ». Ce sont généralement des plantes herbacées, plus rarement ligneuses (bambous).

Comme tous les pollens anémophiles, le pollen des poacées est de forme sphérique ou légèrement ellipsoïdale à ornementations réduites. L'aperture unique (ou pore) est ronde : c'est un des critères des monocotylédones. Le pollen des poacées est de petite taille et léger. La taille est de l'ordre de 40 microns. Pour les céréales, la taille est de 60 à 100 microns.

La famille des pinacées (*Pinaceae*), ou abiétacées, regroupe des plantes gymnospermes ; elle compte 220-250 espèces réparties en 11 genres. Ce sont des arbres ou des arbustes, des régions tempérées, soit à feuilles persistantes en aiguille ou en écailles, soit caduques comme celles des mélèzes. Dans cette famille, les espèces indigènes en France se trouvent parmi les genres *Abies* (les sapins), *Picea* (les épicéas), *Larix* (le mélèze d'Europe), *Pinus* (les pins).

Les pinacées produisent de gros grains de pollen de manière abondante dont la taille est généralement comprise entre 40 et 100 microns. Ils sont dépourvus de pores. Les grains de pollen des pins, sapins, épicéa et cèdre possèdent deux ballonnets qui facilitent leur suspension dans l'air. Les grains de pollen des mélèzes et des douglas sont plus ou moins sphériques et sans ballonnet.

Les plantes à partir desquelles les pollens et pistil sont obtenus sont choisies selon la revendication 1.

De préférence, les plantes utilisées, à partir desquelles les pollens et/ou pistil sont obtenus, sont fraichement récoltées. Les pollens utilisés pour la présente invention peuvent être des pollens récoltés par des insectes (tel que du pollen d'abeille) ou récoltés par une intervention humaine. Le pollen d'abeille par exemple contient du pollen, mais aussi du nectar et de la salive d'abeille. Le pollen récolté via une intervention humaine est dépourvu de tels ingrédients additionnels. De préférence, lesdits pollen des présentes compositions sont obtenus seulement par une intervention humaine. Ceci permet une fois de plus une standardisation du produit final.

La composition pour son utilisation selon la présente invention est avantageusement riche en superoxyde dismutase (SOD), tannins, polyphénols, vitamines, enzymes et oligoéléments, acides aminés, acide gras et minéraux. Par ailleurs, la composition pour son utilisation selon la présente invention ne contient pas d'hormones, tels que les phytoestrogènes.

De façon avantageuse, les extraits de pollen sont obtenus à partir du cytoplasme (partie intérieure de la graine de pollen dépourvue de son enveloppe). Comme l'enveloppe est généralement une source d'allergènes, et un obstacle à la disponibilité des composés du cytoplasme, l'utilisation d'un extrait cytoplasmique de pollen présente un avantage évident comparé à l'utilisation d'un extrait de pollen naturel. De tels extraits cytoplasmiques purifiés de pollen spécifiques, standardisés, ont également une forte teneur en superoxyde dismutase (SOD), tannins, polyphénols, vitamines, enzymes et oligoéléments, acides aminés, acide gras et minéraux, tout comme en protéines bénéfiques et en hydrates de carbone. La concentration de composés bénéfiques dans les différents extraits est largement supérieure à la quantité de composés intéressants dans les pollens à l'état brut.

Il a été montré que l'efficacité d'une composition comprenant des extraits de pollen cytoplasmiques purifiés spécifiques, standardisés, d'un mélange de matières végétales de plusieurs plantes sur l'infertilité, est plus importante en comparaison avec des compositions qui comprennent des extraits d'une seule plante seulement.

La composition pour son utilisation selon l'invention comprend :
- un extrait aqueux de pollen de *Secale cereale* L. ;
- un extrait aqueux de pollen de *Zea mays* L. ;
- un extrait aqueux de pollen de *Pinus silvestris* L. ;
- un extrait aqueux de pollen de *Dactylis glomerata* L. ; et
- un extrait aqueux de pistil de *Zea mays* L., caractérisé en ce que l'extrait de pollen et l'extrait de pistil sont des extraits aqueux dont l'extraction est réalisée à une température inférieure à 45°C.

Préférentiellement, la composition pour son utilisation selon l'invention comprend :
- 45%à 90% d'extrait aqueux de pollen de *Secale cereale* L. en poids du poids total de l'extrait ;
- 1% à 35% d'extrait aqueux de pollen de *Zea mays* L. en poids du poids total de l'extrait ;
- 0,01% à 5% d'extrait aqueux de pollen de *Pinus silvestris* L. en poids du poids total de l'extrait ;
- 3% à 30% d'extrait aqueux de pollen de *Dactylis glomerata* L. en poids du poids total de l'extrait ; et
- 0,1% à 10% d'extrait aqueux de pistil de *Zea mays* L. en poids du poids total de l'extrait.

De préférence, la dose journalière des extraits aqueux de pollen et de pistil de la composition pour son utilisation selon la présente invention est comprise entre 160 mg et 480 mg, plus préférentiellement entre 160 et 320 mg. Cette dose journalière est préférentiellement administrée en 1, 2 ou 3 prises (matin, midi et/ou soir) par exemple sous forme de 1, 2, 3, 4 ou 6 comprimés.

Le poids final du comprimé est préférentiellement compris entre 300 mg et 1000 mg. Plus préférentiellement encore, il est compris entre 325 et 650 mg.

De façon particulièrement avantageuse, le poids final du comprimé est de 650 mg.

La composition pour son utlisation selon l'invention est avantageusement administrée pendant une période d'au moins trois mois, préférentiellement six mois, soit plus de deux cycles de production des spermatozoïdes. Avantageusement, une telle durée de traitement va permettre de tenter de générer la production de suffisamment de spermatozoïdes présentant notamment une bonne mobilité et qualité (volume, pH, nombre, morphologie, vitalité, etc.) par les testicules chez des hommes produisant peu de spermatozoïdes fertiles, de sorte à leur permettre de retrouver une fertilité naturelle ou au moins augmenter sensiblement les chances de trouver des spermatozoïdes fertiles dans l'éjaculat pour une fécondation *in vitro,* et ainsi d'éviter de devoir se tourner vers le don de sperme ou l'adoption pour avoir un enfant.

De façon avantageuse, la composition pour son utilisation selon l'invention permet en outre une l'amélioration du désir sexuel (libido), des dysfonctionnements érectiles ou encore de l'éjaculation. L'amélioration de ces désordres permet d'augmenter également la probabilité d'avoir un enfant.

Divulgué mais pas revendiqué est un complément alimentaire comprenant la composition décrite.

En effet, les pollens ou les préparations de pollen sont généralement consommés comme un complément alimentaire. Ils peuvent être pris dans leur forme rudimentaire, qui sont les pollens en tant que tels, comme poudre sous forme libre ou encapsulée.

La composition pour son utilisation selon l'invention comprend également un milieu physiologiquement acceptable proportionné à un rapport avantage/risque raisonnable, comprenant des excipients connus et couramment utilisés en phytothérapie tels que des liants, agents de désintégration, agents de charge, agents de dispersion, agents agglomérants, lubrifiants, agents mouillants, tensioactifs, émulsifiants, épaississants, agents de coulance, agents aromatisants, agents édulcorants, colorants, agents de pelliculage, stabilisants et/ou conservateurs.

L'homme du métier veillera à choisir ces éventuels excipients et leur quantité de manière à ce qu'ils ne nuisent pas aux propriétés intéressantes des compositions utilisées selon l'invention.

A titre d'exemple d'excipients, on peut citer notamment la cellulose, préférentiellement la cellulose microcristalline, le dioxyde de silicium.

Préférentiellement, la composition pour son utilisation selon l'invention comprend en outre du coenzyme Q10.

En effet, le coenzyme Q10 permet d'atténuer le stress oxydatif dans le liquide séminal et augmente l'activité des enzymes antioxydantes. La publication *"Effect* of *Coenzyme Q10 supplémentation on antioxidant enzymes activity and oxidative stress of séminal plasma: a double-blind randomised clinical trial."* (Andrologia. 2013 Jan 7. doi: 10.1111/and.12062, Nadjarzadeh et al.) indique qu'une supplémentation en coenzyme Q10 chez des hommes infertiles permet d'atténuer le stress oxydatif dans le liquide séminal, d'améliorer la qualité des spermatozoïdes et d'augmenter l'activité des enzymes antioxydantes.

Avantageusement, la composition pour son utilisation selon l'invention comprend en outre au moins un acide aminé, un caroténoïde, un oligoélément, un stéréoisomère de l'inositol et/ou une vitamine et/ou un extrait de racine, pris seuls ou en mélange.

De préférence :
- l'acide aminé est la lysine, la méthionine ou la carnitine, préférentiellement la L-carnitine ;
- le caroténoïde est le lycopène ;
- l'oligoélément est choisi parmi le zinc et le sélénium ;
- le stéréoisomère de l'inositol est le myo-inositol ;
- la vitamine est choisie parmi la vitamine B6, la vitamine B9, la vitamine B12, la vitamine C, la vitamine D et la vitamine E ; et
- l'extrait de racine est un extrait de la racine de maca *(Lepidium meyenii).*

Cette pluralité d'ingrédients spécifiquement choisis et associés selon l'invention permet des corrélations entre ces principes actifs qui agissent en synergie dans la composition pour son utilisation selon l'invention pour générer et optimiser notamment la production et la mobilité des spermatozoïdes chez des hommes hypofertiles ou infertiles, et de manière générale améliorer la qualité et plus particulièrement la vitalité des spermatozoïdes ainsi produits (notamment le pH, nombre, morphologie, vitalité, etc.).

La composition pour son utilisation selon l'invention est administrable par voie orale, en une ou plusieurs formulations identiques ou différentes. Elle se présente sous une forme galénique quelconque normalement utilisée pour une administration orale et notamment sous la forme de gélule, comprimé, capsule, capsule molle, dragée, sachet, tube, flacon, chewing-gum, bille, émulsion, suspension, liquide, solution, ampoule, boisson, sirop, poudre, solide, gel mou, semi-solide.

Avantageusement, la composition est formulée sous forme de comprimé, gélule, capsule, gel mou, semi-solide, solide, liquide ou poudre.

De façon particulièrement avantageuse, la composition pour son utilisation selon l'invention est administrée une fois par jour et se présente sous forme d'un comprimé, dont le poids est préférentiellement de 650 mg.

Généralement, les compositions pour l'utilisation selon l'invention peuvent être obtenues par mélange de différents extraits de pollen et/ou de pistil obtenus séparément par des procédés d'extraction distincts, qui peuvent être identiques ou différents. Ainsi, la composition pour son utilisation selon l'invention peut par exemple comprendre un premier extrait de pollen et de pistil combiné avec un second extrait de pollen. Avantageusement, le premier extrait de pollen et de pistil est un extrait combiné de cytoplasme purifié de pollen et de pistil, contenant une activité élevée d'une enzyme antioxydante, la superoxyde dismutase (SOD). Le second extrait de pollen est un extrait cytoplasmique purifié de pollen pur. Le pollen et les pistils sont sélectionnés et récoltés, séparément et de manière standardisée, parmi les plantes selon la revendication 1. La culture et la récolte des espèces définies sont effectuées dans des champs séparés sous un contrôle-qualité dûment établi conformément aux Bonnes Pratiques Agricoles applicables aux plantes médicinales. Le pollen est sélectionné pour la préparation du second extrait de pollen, tandis que les pollens et pistils sélectionnés sont mélangés d'une manière standardisée pour produire la formulation du premier extrait. Au cours du processus d'extraction, mis en oeuvre conformément aux Bonnes Pratiques de Fabrication, le pollen et les pistils sont traités avec des enzymes pour obtenir l'ouverture germinative. L'extrait est ensuite récupéré par filtration, laissant de côté les enveloppes des grains de pollen qui peuvent être allergisantes. L'extrait est défini par chromatographie liquide haute performance (HPLC) et chromatographie en phase gazeuse (GC) pour garantir la quantité des principes actifs. L'extrait est mélangé dans une formule standardisée. Cette procédure de standardisation donne par exemple des comprimés qui contiennent 100-140 mg du premier extrait de pollen et de pistil et 20-60 mg de du second extrait de pollen.

De façon avantageuse, le Demandeur a développé un procédé alternatif de fabrication simultané d'un extrait de pollen et/ou de pistil, par extractions aqueuses successives.

La maitrise des paramètres de fabrication permet d'homogénéiser et de préserver la qualité des extraits de pollen et/ou de pistil des espèces mises en oeuvre, en empêchant notamment une dénaturation de certaines protéines.

Ainsi, le procédé divulgué mais pas revendiqué permet d'assurer une traçabilité des espèces dans l'extrait de pollen et/ou de pistil.

Par ailleurs, le titre de l'extrait de pollen et/ou de pistil est standardisé en acides aminés à sa dernière étape de fabrication. Par cette maitrise des paramètres de fabrication, la traçabilité et la standardisation du titre en acides aminés sont assurées, permettant une excellente reproductibilité intra-lots encadrée par une spécification de l'extrait.

Divulgué mais pas revendiqué est un procédé de préparation d'un extrait de pollen et de pistil de plante(s) appartenant préférentiellement à la famille des pinacées et/ou des poacées comprenant les étapes successives de :
a) extraction aqueuse de pollen ;
b) extraction aqueuse de pollen et de pistil ;
c) séchage par atomisation des extraits obtenus aux étapes a) et b) ;
d) récupération desdits extraits de pollen et de pistil de plante(s) obtenus en c).

Le Demandeur a pu mettre en évidence que l'étape d'extraction était particulièrement sensible. Ainsi, la température de l'extraction doit être strictement inférieure à 45°C. Au-delà de cette température, un ou plusieurs pollens et pistils détaillés ci-dessus ne sont plus présents (absence de marqueurs spécifiques). La qualité de la composition et/ou son efficacité ne sont donc pas assurées.

Préférentiellement, la température de l'extraction doit être inférieure à 42°C.

La durée de l'étape d'extraction, pour chacun des extraits, est préférentiellement d'au moins 6h, préférentiellement au moins 10h, plus préférentiellement encore au moins 12h.

De plus, le Demandeur a pu mettre en évidence qu'une séparation dans des conditions trop soutenues dégradait le ou les extraits. Ainsi, lorsque l'extraction est couplée à une séparation, celle-ci ne doit pas dépasser les 6000 tours par minute (tr/min), préférentiellement 4500 tr/min, plus préférentiellement encore 2800 tr/min.

Le procédé ainsi développé permet de garantir une bonne traçabilité des pollens et pistils utilisés. En effet, le Demandeur a pu mettre en évidence que les extraits fabriqués selon les procédés de l'art antérieur ne permettent pas de retrouver la présence d'un ou plusieurs marqueurs des pollens et pistils et donc, de facto, de la présence de l'ensemble des extraits de pollens et/ou de pistils dans la composition.

Les compositions de l'art antérieur ne sont donc pas homogènes et ne contiennent pas, dans la composition finale, l'ensemble des extraits de pollens et/ou de pistils attendus.

Le procédé divulgué mais pas revendiqué comprend avantageusement des étapes additionnelles de séparation, de filtration et/ou d'évaporation permettant d'augmenter la concentration de l'extrait final ou d'optimiser la préparation dudit extrait.

Le procédé divulgué mais pas revendiqué présente par ailleurs les avantages suivants :
- il permet une standardisation ;
- il présente une excellente reproductibilité ;
- il permet une réduction des coûts en optimisant et limitant le nombre d'opérations de l'outil industriel.

Il permet également de pouvoir tracer la présence des extraits dans la composition selon l'invention et ainsi assurer sa qualité et par là même son efficacité.

Parmi les avantages permettant de réduire les manipulations et les coûts, on peut citer :
- une planification simplifiée ;
- une occupation des équipements optimisés ;
- des temps improductifs réduits (nettoyage, changement de lot, nombre de validations, etc.) ;
- une gestion du nombre de référence limitée ;
- une diminution des opérations à risque (nettoyage, pesées, procédures de travail, coûts réduits en validation, stabilité, formulation du comprimé simplifiée, risque microbiologique, traçabilité, etc.).

Compte tenu du fait que le procédé de préparation divulgué mais pas revendiqué permet d'obtenir un extrait de pollen et de pistil contenant des marqueurs non indentifiables lors de l'utilisation de procédé de préparation d'extrait selon l'art antérieur, est décrit mais pas revendiqué l'obtention d'un extrait de pollen et de pistil appartenant préférentiellement à la famille des pinacées et/ou des poacées susceptible d'être obtenu selon le procédé de préparation décrit ci-dessus.

De préférence, elle a pour objet un extrait aqueux de pollen et/ou de pistil de *Secale céréale L.,* de *Zea mays L.,* de *Pinus silvestris L.* et/ou de *Dactylis glomerata L.,* susceptible d'être obtenu selon le procédé de préparation comprenant les étapes suivantes de :
a) extraction à l'eau de pollens de *Secale céréale L.,* de *Zea mays L.,* de *Pinus silvestris L.* et/ou de *Dactylis glomerata L.,* à une température inférieure à 45°C afin d'obtenir un premier extrait ;
b) extraction à l'eau de pollens et de pistils de *Zea mays L.,* à une température inférieure à 45°C afin d'obtenir un second extrait ;
c) mélange du premier et du second extraits obtenus en a) et b) ;
d) séchage par atomisation du mélange obtenu en c) ;
e) récupération du mélange d'extraits de pollen et de pistil obtenu en d).

Préférentiellement, la température de l'extraction, pour chacun des extraits, doit être inférieure à 42°C.

Plus préférentiellement encore, elle a pour objet un extrait aqueux de pollen et/ou de pistil de *Secale céréale L.,* de *Zea mays L.,* de *Pinus silvestris L.* et/ou de *Dactylis glomerata L.* tel que décrit ci-dessus, pour son utilisation dans le traitement et/ou la prévention de l'infertilité.

Le Demandeur a pu mettre en évidence que la production d'une composition à partir d'extraits de plantes requiert souvent des étapes de production spécifiques telles que par exemple le séchage par atomisation, la granulation. Ces étapes peuvent avoir un impact sur la composition finale et ses constituants telles que les protéines. Certaines protéines qui sont présentes dans la matière végétale brute sont susceptibles, à cause de la nature spécifique du procédé de fabrication (typiquement le séchage par atomisation), de perdre leur activité ou d'être perdues. Le Demandeur a notamment pu mettre en évidence que l'utilisation d'un procédé de séchage par atomisation optimisé (différence de température de 50°C entre la température d'entrée et celle de sortie) évitait la dénaturation de protéines cibles concernées. Le Demandeur a également trouvé que la présence dans la composition finale d'au moins une de ces protéines ou peptides dérivés depuis ces protéines cibles permet d'assurer la qualité de la composition. Les extraits utilisés dans la composition sont ainsi optimaux et de qualité pharmaceutique, l'activité de la composition n'étant pas altérée. Par ailleurs, en fournissant un contrôle qualité adéquat, une meilleure uniformité du contenu peut être assurée, ce qui est important quand on traite des patients pour des symptômes spécifiques et/ou des inconforts.

De plus, la composition est fortement reproductible entre différents lots (en termes de qualité et d'ingrédients présents), ce qui permet un contrôle qualité, une standardisation, une traçabilité des lots, et un profilage de protéines reproductibles. Elle est ainsi en conformité avec les bonnes pratiques de fabrication des produits pharmaceutiques et des compléments alimentaires sur le marché (produits botaniques de qualité pharmaceutique). Cette reproductibilité donne également la garantie que chaque nouveau lot d'extrait de pollen et/ou de pistil satisfait aux spécifications établies et de ce fait à la même activité physiologique que les lots utilisés pour les études cliniques. Le procédé de fabrication permet d'obtenir le composé bénéfique recherché.

Dans les compositions pour l'utilisation selon l'invention la présence d'un ou plusieurs marqueurs de protéines est détectable, selon lesquelles ces marqueurs de protéines sont une bonne indication de la qualité de la composition finale. Ces marqueurs peuvent être également utilisés pour la standardisation des compositions.

Lesdits marqueurs ou traceurs sont avantageusement des protéines ou des peptides dérivés de protéines, lesdites protéines étant choisies parmi le groupe de l'oxydase de réticuline, de l'endochitinase A, de la bêta-1,3-glucanase, de l'exopolygalacturonase, de la protéine de transfert lipidique non spécifique ou de toute combinaison de ces dernières.

Selon l'invention, la présence de marqueurs spécifiques de protéines ou de peptides provenant de ces protéines (marqueurs) peut être confirmée dans la composition finale, lesdits marqueurs étant révélateurs de la qualité de la composition et peuvent donc être utilisés pour la standardisation et le contrôle qualité.

En effet, le Demandeur a pu mettre en évidence que la présence de certains traceurs ou marqueurs (protéine ou peptide dérivé de ladite protéine) permettaient de confirmer la présence de certains extraits de pollen et/ou de pistil dans la composition finale.

Ainsi, le Demandeur a pu démontrer que les protéines suivantes ou peptides dérivés desdites protéines permettent la traçabilité des pollens et/ou pistils suivants :
- l'allergène de pollen Sec 4 (**Secale cereale**) (Q5TIW8 et Q5TIW7), la glucanendo - 1,3-beta-D-glucosidase (Q1EM97) permettent par exemple de tracer la présence du pollen *Secale cereale L.* ;
- l'oxydase de réticuline (Reticulase Oxidase) (B6T5D7), la beta-1,3-glucanase (E1AFV5), la pectinesterase (B6UCK8), la protéine Extensin-like (Q9SPM0), l'exopolygalacturonase (PGLR2), la beta-amylase (Q9SYS1), la chitinase (D0EM57) permettent par exemple de tracer les pistils et pollens de *Zea mays L.* ;
- l'allergène de pollen Lol p 4 permet par exemple de tracer la présence du pollen *Dactylis glomerata L.* ; et
- la protéine de transfert lipidique permet par exemple de tracer la présence du pollen *Pinus sylvestris L. et*/*ou Zea mays L..*

Lesdites protéines ou lesdits peptides dérivés desdites protéines présents dans les extraits finaux et de fait dans la composition pour son utilisation selon l'invention sont préférentiellement choisis parmi le groupe de l'oxydase de réticuline (Reticulase Oxidase), de l'endochitinase A, de la bêta-1,3-glucanase, de l'exopolygalacturonase, de la protéine de transfert lipidique non spécifique ou de toute combinaison de ce qui précède.

Les extraits de la composition pour son utilisation selon l'invention comprennent avantageusement au moins une deuxième protéine ou un peptide dérivé de ladite deuxième protéine choisis parmi le groupe de l'allergène de pollen Lol p 4 de *Dactylis glomerata L.,* de l'allergène de pollen Sec 4 (**Secale cereale**), la glucanendo-1,3-beta-D-glucosidase, la beta-amylase, la chitinase ou toute combinaison de ces dernières.

Le Demandeur a pu mettre en évidence que l'absence d'un ou plusieurs de ces marqueurs ou traceurs, spécifiques de certains pollens et/ou pistils, dans les extraits contenant *a priori* lesdits pollens et/ou de pistils et de fait dans les compositions comprenant a priori de tels extraits était des indicateurs qualitatifs des produits.

Ainsi, l'absence d'un marqueur spécifique d'un pollen et/ou d'un pistil donné indique que l'extrait ou la composition ne comprend pas ledit pollen et/ou pistil, ou qu'ils ont été dénaturés, rendant ainsi la composition comprenant au moins un tel extrait de pollen et/ou de pistil dégradé, inefficace ou, à tout le moins, moins efficace.

L' amélioration de la qualité du sperme se manifeste notamment sur les paramètres suivants :
- le volume du sperme ;
- le pH du sperme ;
- la concentration des spermatozoïdes ;
- la mobilité totale des spermatozoïdes ;
- la densité du sperme ;
- la vitalité et la morphologie des spermatozoïdes ; ou
- la présence de leucocyte.

Selon un mode particulier de réalisation de l'invention, la composition est utilisée dans le traitement et/ou la prévention de l'infertilité chez des hommes non associée à une inflammation d'origine microbactérienne telle qu'une prostatite. Ainsi, aucun antibiotique n'est associé en combinaison avec la composition pour son utilisation selon l'invention.

En effet, l'infertilité masculine au sens de l'invention s'entend comme pouvant être également une infertilité non liée à la prostate.

La présente invention va maintenant être illustrée au moyen des exemples suivants :

### Exemple 1 : Compositions selon l'invention

i. Composition A :
Forme galénique : Comprimé
Poids du comprimé : environ 380 mg
Extrait aqueux de pollen : environ 40% en poids du poids total de la composition

**Tableau 1 : Ingrédients de l'extrait exprimés en poids du poids total de l'extrait final de pollen :**

| **Ingrédients** | **Quantité %** |
|---|---|
| *Secale cereale* L. Pollen | 70-80 |
| *Zea mays* L. Pollen | 15-40 |
| *Dactylis alomerata* L. Pollen | 5-10 |

D'autres ingrédients dans le produit final sont par exemple : la cellulose microcristalline, le dioxyde de silicium, le stéarate de magnésium et/ou des agents de pelliculage. Le talc et la gomme-laque ont été utilisés en tant qu'agent de pelliculage. La dose quotidienne recommandée est de deux comprimés par jour, à prendre le matin ou le soir.

L'extrait de pollen du comprimé ci-dessus a été obtenu en utilisant un procédé classique de préparation des extraits de pollen et/ou de pistil.

### ii. Composition B :

Forme galénique : Gélule
Poids du comprimé : environ 380 mg ou 640 mg
Extrait aqueux de pollen : environ 40% en poids du poids total de la composition

**Tableau 2 : Ingrédients de l'extrait exprimés en poids du poids total de l'extrait final de pollen et de pistil :**

| **Ingrédients** | **Quantité %** |
|---|---|
| *Zea mays* L. Pollen | 15-80 |
| *Pinus sylvestris* L. Pollen | 0,05 à 25,00 |
| *Zea mays* L. Pistil | 1-15 |

D'autres ingrédients dans le produit final sont par exemple : la cellulose microcristalline, le dioxyde de silicium, le stéarate de magnésium et/ou des agents de pelliculage. Le talc et la gomme-laque ont été utilisés en tant qu'agent de pelliculage. La dose quotidienne recommandée est d'un ou deux comprimés par jour, à prendre le matin ou le soir.

L'extrait de pollen et de pistil du comprimé ci-dessus a été obtenu en utilisant un procédé classique de préparation des extraits de pollen et/ou de pistil.

### iii. Composition C :

Forme galénique : Comprimé
Poids du comprimé : environ 380 mg ou 640 mg
Extrait aqueux de pollen : environ 40% en poids du poids total de la composition

**Tableau 3 : Ingrédients de l'extrait exprimés en poids du poids total de l'extrait final de pollen et de pistil :**

| **Ingrédients** | **Quantité %** |
|---|---|
| *Secale cereale* L. Pollen | 45-90 |
| *Zea mays* L. Pollen | 1-35 |
| *Pinus sylvestris* L. Pollen | 0,01 à 5,00 |
| *Dactylis glomerata* L. Pollen | 3-30 |
| *Zea mays* L. Pistil | 0,1-10 |

D'autres ingrédients dans le produit final sont par exemple : la cellulose microcristalline, le dioxyde de silicium, le stéarate de magnésium et/ou des agents de pelliculage. Le talc et la gomme-laque ont été utilisés en tant qu'agent de pelliculage. La dose quotidienne recommandée est de deux comprimés par jour, à prendre le matin ou le soir.

L'extrait de pollen et de pistil du comprimé ci-dessus a été obtenu en utilisant un procédé classique de préparation des extraits de pollen et/ou de pistil.

### iv. Composition D :

Forme galénique : Comprimé
Poids du comprimé : environ 380 mg ou 640 mg
Extrait aqueux de pollen : environ 40% en poids du poids total de la composition

**Tableau 4 : Ingrédients de l'extrait exprimés en poids du poids total de l'extrait final de pollen et de pistil :**

| **Ingrédients** | **Quantité %** |
|---|---|
| *Secale cereale* L. Pollen | 45-90 |
| *Zea mays* L. Pollen | 1-35 |
| *Pinus sylvestris* L. Pollen | 0,01 à 5,00 |
| *Dactylis glomerata* L. Pollen | 3-30 |
| *Zea mays* L. Pistil | 0,1-10 |

D'autres ingrédients dans le produit final sont par exemple : la cellulose microcristalline, le dioxyde de silicium, le stéarate de magnésium et/ou des agents de pelliculage. Le talc et la gomme-laque ont été utilisés en tant qu'agent de pelliculage. La dose quotidienne recommandée est de deux comprimés par jour, à prendre le matin ou le soir.

L'extrait de pollen et de pistil du comprimé ci-dessus a été obtenu en utilisant le procédé détaillé à l'exemple 2 ci-dessous,

### v. Composition E :

Forme galénique : Comprimé
Poids du comprimé : environ 650 mg
Extrait aqueux de pollen : environ 40% en poids du poids total de la composition

**Tableau 5 : Ingrédients de l'extrait exprimés en poids du poids total de l'extrait final de pollen et de pistil :**

| **Ingrédients** | **Quantité %** |
|---|---|
| *Secale cereale* L. Pollen | 45-90 |
| *Zea mays* L. Pollen | 1-35 |
| *Pinus sylvestris* L. Pollen | 0,01 à 5,00 |
| *Dactylis glomerata* L. Pollen | 3-30 |
| *Zea mays* L. Pistil | 0,1-10 |

Le produit final intègre en outre environ 2 à 10 mg de zinc et d'autres ingrédients comme par exemple : la cellulose microcristalline, le dioxyde de silicium, la vitamine E, le stéarate de magnésium et/ou des agents de pelliculage. Le talc et la gomme-laque ont été utilisés en tant qu'agent de pelliculage. La dose quotidienne recommandée est d'un comprimé parjour, à prendre le matin ou le soir.

L'extrait de pollen et de pistil du comprimé ci-dessus a été obtenu en utilisant le procédé détaillé à l'exemple 2 ci-dessous.

La présence de l'Oxydase de réticuline, de l'endochitinase A, de la bêta-1,3-glucanase, de l'exopolygalacturonase, et de la protéine de transfert lipidique non spécifique a été conformée par CPL-SM/SM.

L'invention concern une composition comprenant :
- un extrait aqueux de pollen de Secale céréale L. ;
- un extrait aqueux de pollen de Zea mays L. ;
- un extrait aqueux de pollen de Pinus silvestris L. ;
- un extrait aqueux de pollen de Dactylis glomerata L. ; et
- un extrait aqueux de pistil de Zea mays L.,
pour son utilisation dans le traitement et/ou la prévention de l'infertilité, chez des hommes présentant une oligospermie, une cryptozoospermie, une azoospermie, une asthénospermie, une oligoasthénospermie et/ou une tératospermie, caractérisé en ce que l'extrait de pollen et l'extrait de pistil sont des extraits aqueux dont l'extraction est réalisée à une température inférieure à 45°C.

### Example 2 : Production d'un extrait séché par atomisation

Le procédé de préparation de l'extrait décrit ici est une extraction aqueuse permettant une sélection maitrisée des protéines hydrosolubles d'intérêt. La chronologie des opérations, la maitrise des paramètres de fabrication permet de préserver des protéines ou peptides spécifiques caractéristiques des espèces mises en oeuvre. Ceci assure une traçabilité des espèces dans l'extrait. Le titre de l'extrait est avantageusement standardisé en acides aminés à sa dernière étape de fabrication, lors de la nébulisation. Par cette maitrise des paramètres de fabrication, une traçabilité, une standardisation du titre en acides aminés et une reproductibilité intra-lots encadrée par une spécification de l'extrait sont assurées.

### i. Préparation des extraits de pollen et de pistils selon le procédé innovant :

La période appropriée de récolte est :
- juin-juillet pour le pollen de seigle (*Secale cereale L*.) et le pollen de dactyle (*Dactylis glomerata L*.) ;
- juillet-septembre pour le pollen et le pistil de maïs (*Zea mays L*.) ;
- mai-juin pour le pollen de pin (*Pinus silvestris L*.).

Le pollen de pin est récolté à l'état sauvage, les autres pistils et pollen sont issus de cultures agricoles.

Les pollens et pistils sont séchés.

Le procédé peut être caractérisé par les étapes principales suivantes :
a) extraction aqueuse (préférentiellement eau et/ou tensioactif) de pollen ;
b) extraction aqueuse (préférentiellement eau et/ou tensioactif) de pollen et de pistil ;
c) séchage par atomisation des extraits obtenus aux étapes a) et b) ci-dessus ; et
d) récupération desdits extraits de pollen et de pistil de plante(s) obtenus en c).

De façon surprenante, le Demandeur a pu identifier qu'en modifiant les conditions opératoires du procédé de préparation des extraits destinés à l'élaboration des compositions selon l'invention, il était possible d'augmenter la qualité des extraits.

### ii. Détails des étapes principales du procédé préféré selon l'invention :

### a. Etapes d'extraction :

Le Demandeur a pu mettre en évidence que la température des extractions devait être comprise entre 30°C - 45°C.

De préférence, l'extraction est réalisée sous agitation continue, pendant 12 à 90 heures.

### b. Etape de séchage par atomisation du mélange d'extraits :

Les différents extraits, préférentiellement préalablement évaporés, filtrés et/ou décantés, sont mélangés afin d'avoir une substance sèche entre 30% et 50%.

La température de départ de l'étape de séchage par atomisation est comprise entre 138°C et 168°C.

### Exemple 3 : Comparatif des compositions C et D issues de deux procédés différents :

La composition D obtenue selon le procédé décrit ci-dessus (exemple 2) est comparée avec la composition C obtenue selon le procédé de l'art antérieur, pour lequel la température d'extraction est strictement supérieure à 45°C.

Le Demandeur a pu constater que la modification de la température d'extraction et des conditions de séparation physiques, ont une incidence sur le résultat des tests d'identification.

Dans les lots d'extraits obtenus selon le procédé de l'art antérieur, on ne retrouve pas la protéine Q5TIW3 (marqueur des pollens de *Dactylis glomerata L*.) alors qu'elle est présente dans les lots d'extraits obtenus selon le procédé décrit à l'exemple 2 ci-dessus.

Il n'y a pas de traçabilité des espèces avec l'ancien procédé.

La mise au point du procédé est une réponse à une exigence de traçabilité et de reproductibilité, tout en intégrant les contraintes économiques industrielles. La recherche des marqueurs/traceurs dans les extraits fabriqués selon les anciennes méthodes et ceux fabriqués avec le procédé décrit mais pas divulgué montre que les procédés de l'art antérieur ne permettent pas d'assurer la traçabilité des espèces mises en oeuvre.

**Tableau 6 : marqueurs ou traceurs présents dans deux compositions dont les extraits sont obtenus par deux procédés différents :**

| | | |
|---|---|---|
| Espèce | Marqueurs des extraits issus du procédé selon l'exemple 9 (T°<45°C) | Marqueurs des extraits issus du procédé selon l'art antérieur (T°C>45°) |
| Pollens de Secale cereale L. | Q5TIW8 - allergène de pollen Sec 4 | Q5TIW8 - allergène de pollen Sec 4 |
| Pollens et pistils de Zea Mays L. | B6T5D7 - Reticulase oxidase | B6T5D7 - Reticulase oxidase |
| | E1AFV5 - Beta-13-glucanase | E1AFV5 non identifié |
| Pollens de Dactylis glomerata L. | Q5TIW3 allergène de pollen Lol p4 | Non identifié |
| Pollens de Pinus sylvestris L. | Protéine de transfert lipidique, réaction immunologique positive | Protéine de transfert lipidique, réaction immunologique positive |

### Exemple 4 : Efficacité des compositions pour l'utilisation selon l'invention :

### i. Compositions testées :

Dans le présent exemple, les effets de compositions dont les extraits de pollen et de pistil sont obtenus selon le procédé objet de l'invention (composition D ci-dessus) ont été comparées avec des compositions selon l'art antérieur.

### ii. Déroulé de l'étude observationnelle :

Cette étude a pour objectif d'évaluer l'impact d'un traitement de six mois par la composition D ou par d'autres compositions (sensiblement de même forme galénique, de même poids et de même concentration de l'extrait de pollen dans la composition) selon l'art antérieur comprenant des extraits de pollen (EA10P5, EA5P2, *etc*.) sur les paramètres spermatiques principaux (*cf*. ci-dessous) dans une population d'hommes consultant pour un problème d'infertilité et présentant plus particulièrement une oligospermie, une asthénospermie ou une oligoasthénospermie.

Nous nous focalisons sur l'analyse de l'impact d'un tel traitement notamment au niveau de la numération et/ou de la mobilité spermatique chez les patients inclus dans l'étude.

A moins qu'il ne soit spécifié autrement, lors de la visite de début de traitement, les compositions sont remises au patient pour un traitement d'une prise quotidienne pendant six mois. La dose quotidienne recommandée est de deux comprimés par jour, à prendre le matin ou le soir. Les prises débutent le jour même.

Au cours de la visite de fin de protocole, un spermogramme post traitement est réalisé. L'observance est également évaluée.

De façon surprenante, il a été montré qu'une administration pendant trois mois de la composition D améliore davantage la qualité du sperme qu'une composition selon l'art antérieur, notamment en ce qui concerne la mobilité générale du sperme.

En effet, il a été observé que la mobilité était améliorée d'au moins 15%, tandis qu'elle n'est améliorée que d'environ 10% avec une composition comprenant du pollen EA10P5.

De même, il a été montré que la composition D améliore la vitalité du sperme, alors qu'elle est diminuée d'environ 15% avec une composition comprenant du pollen EA10P5.

Par ailleurs, le Demandeur a pu observer que la composition D objet de l'invention améliore également certains désordres tels que la dysfonction sexuelle, la baisse de libido, la dysfonction érectile, l'éjaculation précoce ou retardée.

Le Demandeur a également comparé les effets sur les paramètres du sperme de la composition D objet de l'invention avec une autre composition comprenant du pollen de palme (*Phoenix dactylifera*). Il a ainsi pu montrer que la composition D améliore de plus de 60% le nombre de spermatozoïdes par ml de sperme après six mois de traitement, tandis qu'elle n'est améliorée que d'environ 50% avec une composition équivalente comprenant du pollen de palme (*Phoenix dactylifera*).

Enfin, le Demandeur a comparé les différents paramètres d'un spermogramme de 25 patients avant et après traitement par la composition D pendant une période de six mois.

Le Demandeur a pu observer que les paramètres du spermogramme étaient améliorés, il s'agit notamment des paramètres suivants :
- Volume
- pH
- Nombre
- Mobilité
- Morphologie
- Vitalité
- Leucocytes

L'ensemble de ces études met donc en évidence un effet potentialisé inattendu chez une population de patients avec l'administration de la compositions D selon l'invention qui augmente sensiblement les chances de trouver des spermatozoïdes fertiles dans l'éjaculat en permettant de générer la production de spermatozoïdes mobiles chez des hommes hypofertiles voire infertiles présentant à la fois une oligospermie et une asthénospermie, et ne produisant donc pas ou substantiellement pas de spermatozoïdes fertiles.

L'ensemble des patients évalués dans le cadre de l'étude réalisée répondant favorablement au traitement avec la composition D selon l'invention produisent désormais une concentration suffisante de spermatozoïdes présentant une mobilité adéquate par ml d'éjaculat pour envisager une fécondation naturelle, et ainsi d'éviter de devoir se tourner vers le don de sperme ou l'adoption pour avoir un enfant.

### Exemple 5 : Résultats sur les paramètres du spermogramme obtenus après le traitement par la composition E selon l'invention sur des patients infertiles :

Pendant la première visite d'inclusion les patients réalisent un spermogramme avant traitement. En début de traitement, le produit leur est est remis pour un traitement d'une prise quotidienne pendant trois mois. La dose quotidienne recommandée est d'un comprimé par jour, à prendre le matin ou le soir. Les prises débutent le jour même. Au cours de la visite de fin de protocole, un spermogramme après traitement est réalisé. L'observance est également évaluée. Les conditions de prélèvement sont strictes. Le recueil de sperme se fait par masturbation. Le sperme est recueilli après deux à quatre jours d'abstinence sexuelle, après avoir fait une désinfection soigneuse du gland, et en dehors d'une période de fièvre.

Les résultats comparatifs (avant et après traitement) sont détaillés ci-dessous :
i. Informations concernant le(s) patient(s) dont les résultats sont repris au tableau 7 ci-dessous:
- non-fumeur
- varicocèle gauche de grade 2/3 (La varicocèle se caractérise par la dilatation d'une veine (varice) au niveau du cordon spermatique, cordon fibreux situé dans les bourses au-dessus de chaque testicule, et les reliant chacun au scrotum)
- pas de prise d'autre traitement
- pas de supplémentation vitaminique à l'instauration du traitement

**Tableau 7 :**

| **PARAMETRES** | **Normes OMS 2010** | **Avant traitement** | **Apres traitement** |
|---|---|---|---|
| volume | >1.5 ml | >1.5 ml | >1.5 ml |

| pH | 7.2-8 | 7.2-8 | 7.2-8 |
|---|---|---|---|
| Nombre | 15 million /ml | 10 million /ml | pas de modification |
| Mobilité | > 40% mobilité totale | 26 % mobilité totale | 35% mobilité totale |
| | > 32% mobilité progressive | | |
| Morphologie | >4% selon Krueger | >4% selon Krueger | >4% selon Krueger |
| Vitalité | > 58% | > 58% | > 58% |
| leucocytes | <1 million / ml | <1 million / ml | <1 million / ml |

### Conclusion par rapport aux résultats présentés au tableau 7 :

Avant traitement, la concentration en spermatozoïdes est basse (10 millions/ml) et l'on observe une hypo-mobilité à 26%. Les autres paramètres sont normaux. Après traitement, la concentration en spermatozoïdes reste inchangée, mais la mobilité est augmentée à 35%. Aucun effet indésirable n'est rapporté.
ii. Informations concernant le(s) patient(s) dont les résultats sont repris au tableau 8 ci-dessous:
- fumeur, moins de 20 paquets par jour ;
- pas de comorbidité ;
- pas de prise d'autre traitement ;
- pas de supplémentation vitaminique à l'instauration du traitement.

**Tableau 8 :**

| **PARAMETRES** | **Normes OMS 2010** | **Avant traitement** | **Apres traitement** |
|---|---|---|---|
| volume | >1.5 ml | >1.5 ml | pas de modification |
| pH | 7.2-8 | 7.2-8 | pas de modification |
| Nombre | 15 million /ml | < 1 million /ml | < 1 million/ml |
| Mobilité | > 40% mobilité totale | > 40% mobilité totale | pas de modification |
| | > 32% mobilité progressive | | |
| | | > 32% mobilité progressive | |
| Morphologie | >4% selon Krueger | >4% selon Krueger | pas de modification |
| Vitalité | > 58% | > 58% | pas de modification |
| leucocytes | <1 million / ml | <1 million / ml | pas de modification |

### Conclusion par rapport aux résultats présentés au tableau 8 :

Avant traitement, le patient présente une cryptozoospermie. Après traitement, la cryptozoospermie (concentration inférieure à 1 million /ml) n'a pas été améliorée. Aucun effet indésirable n'est rapporté.
iii. Informations concernant le(s) patient(s) dont les résultats sont repris au tableau 9 ci-dessous:
- fumeur, moins de 20 paquets par jour ;
- pas de comorbidité
- pas de prise d'autre traitement
- pas de supplémentation vitaminique à l'instauration du traitement

**Tableau 9 :**

| **PARAMETRES** | **Normes OMS 2010** | **Avant traitement** | **Apres traitement** |
|---|---|---|---|
| volume | >1.5 ml | >1.5 ml | >1.5 ml |
| pH | 7.2-8 | 7.2-8 | 7.2-8 |
| Nombre | 15 million /ml | 10 millions/ml | pas de modification |
| | >39 millions | 40 millions | |
| Mobilité | > 40% mobilité totale | 27% mobilité totale | 32% mobilité totale |
| | > 32% mobilité progressive | | |
| Morphologie | >4% selon Krueger | 1% | 4% |
| Vitalité | > 58% | 52% | 55% |
| leucocytes | <1 million / ml | <1 million / ml | <1 million / ml |

### Conclusion par rapport aux résultats présentés au tableau 9 :

Avant traitement, le patient présente une numération normale-basse à 40 millions de spermatozoïdes par éjaculat. La concentration en spermatozoïdes est basse, à 10 millions/ml. La mobilité des spermatozoïdes est également basse, à 27%. La vitalité est d'environ 52% et la morphologie est anormale à 1% de formes typiques.

Après traitement, on observe une :
- une amélioration de la mobilité à 32 % et de la vitalité à 55% ; et
- une amélioration de la morphologie 4% de formes normales.

En revanche, la numération et concentration restent inchangées. Aucun effet indésirable n'est rapporté.

### iv. Informations concernant le(s) patient(s) dont les résultats sont repris au tableau 10 ci-dessous:

- Entre 20-50 ans
- Sujets en bonne santé avec un régime alimentaire équilibré
- Exclusion d'azoospermie et stérilité excrétoire
- non-fumeur
- varicocèle gauche de grade 1 (La varicocèle se caractérise par la dilatation d'une veine (varice) au niveau du cordon spermatique, cordon fibreux situé dans les bourses au-dessus de chaque testicule, et les reliant chacun au scrotum)
- pas de comorbidité
- pas de prise d'autre traitement
- pas de supplémentation vitaminique à l'instauration du traitement

**Tableau 10 :**

| **PARAMETRES** | **Normes OMS 2010** | **Avant traitement** | **Apres traitement** |
|---|---|---|---|
| volume | >1.5 ml | 1,8 ml | 2,5 ml |
| pH | 7,2 - 8 | 7,2 - 8 | 8,1 |
| Nombre | 15 million /ml | 1000 /ml | 0,6 M/ml |
| | >39 millions | 15 M | |
| Mobilité | > 40% mobilité totale | 7% progressive | 46% totale |
| | > 32% mobilité progressive | | 28% progressive |
| leucocytes | <1 million / ml | - | 2% |

Par ailleurs, le patient a évalué le produit comme repris au tableau 11 ci-dessous :

**Tableau 11 : questionnaire de satisfaction remis au patient :**

| | | |
|---|---|---|
| Comment jugez-vous l'efficacité de ce produit sur l'amélioration de paramètres du spermogramme ? | 0 = Très efficace | |
| | 1 = Plutôt efficace | x |
| | 2 = Peu efficace | |
| | 3 = Tout à fait inefficace | |
| Comment jugez-vous l'acceptabilité de ce produit (en termes de contrainte de prise, de gout éventuel des comprimés, etc) ? | 0= Très bonne | x |
| | 1= Bonne | |
| | 2= Moyenne | |
| | 3= Mauvaise | |
| Accepteriez-vous d'utiliser régulièrement ce produit ? | 1 = Oui nettement | |
| | 2 = Oui à la rigueur | x |
| | 3 = Non pas du tout | |

### Conclusion par rapport aux résultats présentés au tableau 10 :

Avant traitement, le patient présente une concentration en spermatozoïdes basse, à 1000/ml. La mobilité des spermatozoïdes est également basse, à 7%.

Après traitement, on observe une :
- une augmentation du volume ;
- une augmentation importante de la concentration en spermatozoïdes à 0,6M/ml
- une large amélioration de la mobilité à 46 % totale et 28% progressive.

Les patients considère le traitement plutôt efficace. Aucun effet indésirable n'est rapporté.
v. Informations concernant le(s) patient(s) dont les résultats sont repris au tableau 12 ci-dessous:
- non-fumeur
- Entre 20-50 ans
- Sujets en bonne santé avec un régime alimentaire équilibré
- Exclusion d'azoospermie et stérilité excrétoire
- pas de prise d'autre traitement
- pas de comorbidité
- pas de supplémentation vitaminique à l'instauration du traitement

**Tableau 12 :**

| **PARAMETRES** | **Normes OMS 2010** | **Avant traitement** | **Apres traitement** |
|---|---|---|---|
| volume | >1.5 ml | 4 ml | 3 ml |
| pH | 7,2 - 8 | 7,8 | 7,8 |
| Nombre | 15 million /ml | 17 M/ml | 40 M/ml |
| | >39 millions | | |
| Mobilité | > 40% mobilité totale | 50% totale | 50% totale |
| | > 32% mobilité progressive | 41% progressive | 41% progressive |
| Morphologie | >4% selon Krueger | 2% | 1% |
| Vitalité | > 58% | 75% | 79% |
| leucocytes | <1 million / ml | 1% | 2% |

Par ailleurs, le patient a évalué le produit comme repris au tableau 13 ci-dessous :

**Tableau 13 : questionnaire de satisfaction remis au patient :**

| | | |
|---|---|---|
| Comment jugez-vous l'efficacité de ce produit sur l'amélioration de paramètres du spermogramme ? | 0 = Très efficace | |
| | 1 = Plutôt efficace | x |
| | 2 = Peu efficace | |
| | 3 = Tout à fait inefficace | |
| Comment jugez-vous l'acceptabilité de ce produit (en termes de contrainte de prise, de goût éventuel des comprimés, etc) ? | 0= Très bonne | x |
| | 1= Bonne | |
| | 2= Moyenne | |
| | 3= Mauvaise | |
| Accepteriez-vous d'utiliser régulièrement ce produit ? | 1 = Oui nettement | x |
| | 2 = Oui à la rigueur | |
| | 3 = Non pas du tout | |

### Conclusion par rapport aux résultats présentés au tableau 12 :

Avant traitement, le patient présente une concentration en spermatozoïdes faible, à 15M/ml.

Après traitement, on observe une :
- une augmentation importante de la concentration en spermatozoïdes à 40M/ml
- une faible amélioration de la vitalité à 79% est également observée.

Aucun effet indésirable n'est rapporté.

## Revendications

1. Composition comprenant :
- un extrait aqueux de pollen de *Secale cereale* L. ;
- un extrait aqueux de pollen de *Zea mays* L. ;
- un extrait aqueux de pollen de *Pinus silvestris* L. ;
- un extrait aqueux de pollen de *Dactylis glomerata* L. ; et
- un extrait aqueux de pistil de *Zea mays* L.,
pour son utilisation dans le traitement et/ou la prévention de l'infertilité, chez des hommes présentant une oligospermie, une cryptozoospermie, une azoospermie, une asthénospermie, une oligoasthénospermie et/ou une tératospermie, **caractérisé en ce que** l'extrait de pollen et l'extrait de pistil sont des extraits aqueux dont l'extraction est réalisée à une température inférieure à 45°C.

2. Composition pour son utilisation selon la revendication 1, **caractérisée en ce qu'**elle est utilisée dans le traitement de l'infertilité chez des hommes présentant une asthénospermie et/ou une tératospermie.

3. Composition pour son utilisation selon l'une des revendications précédentes, **caractérisée en ce qu'**elle comprend en outre du coenzyme Q10.

4. Composition pour son utilisation selon l'une des revendications précédentes, **caractérisée en ce qu'**elle comprend en outre au moins un acide aminé, un caroténoïde, un oligoélément, une vitamine et/ou un extrait de racine, pris seuls ou en mélange.

5. Composition pour son utilisation selon la revendication 4, **caractérisée en ce que** :
- l'acide aminé est la lysine, la méthionine ou la carnitine, préférentiellement la L-carnitine ;
- le caroténoïde est le lycopène ;
- l'oligoélément est choisi parmi le zinc et le sélénium ;
- la vitamine est choisie parmi la vitamine B6, la vitamine B9, la vitamine B12, la vitamine C, la vitamine D et la vitamine E ; et
- l'extrait de racine est un extrait de la racine de maca (*Lepidium meyenii*).

6. Composition pour son utilisation selon l'une des revendications 4 et 5, **caractérisée en ce qu'**elle comprend un oligoélément qui est le zinc.

7. Composition pour son utilisation selon l'une des revendications précédentes, **caractérisée en ce qu'**elle est administrée par voie orale.

8. Composition pour son utilisation selon la revendication 7, **caractérisée en ce qu'**elle est formulée sous forme de comprimé, gélule, capsule, gel mou, semi-solide, solide, liquide ou poudre.

9. Composition pour son utilisation selon la revendication 8, **caractérisée en ce qu'**elle est administrée 1 fois par jour et **en ce qu'**elle se présente sous forme d'un comprimé, dont le poids est préférentiellement de 650 mg.

10. Composition pour son utilisation selon l'une des revendications précédentes, **caractérisée en ce qu'**elle est administrée pendant une période d'au moins trois mois.

11. Composition pour son utilisation selon l'une des revendications précédentes, dans le traitement et/ou la prévention de l'infertilité non associée à une inflammation d'origine microbactérienne telle qu'une prostatite.

## Patentansprüche

1. Zusammensetzung, umfassend:
- einen wässrigen Extrakt aus Pollen von *Secale cereale* L.;
- einen wässrigen Extrakt aus Pollen von *Zea mays L.;*
- einen wässrigen Extrakt aus Pollen von *Pinus silvestris* L.;
- einen wässrigen Extrakt aus Pollen von *Dactylis glomerata* L.; und
- einen wässrigen Extrakt aus einem Stempel von *Zea mays* L.,
zur Verwendung bei der Behandlung und/oder der Prävention von Unfruchtbarkeit bei Männern, die Oligospermie, Kryptozoospermie, Azoospermie, Asthenospermie, Oligoasthenospermie und/oder Teratospermie aufweisen, **dadurch gekennzeichnet, dass** der Pollenextrakt und der Stempelextrakt wässrige Extrakte sind, deren Extraktion bei einer Temperatur von weniger als 45 °C durchgeführt wird.

2. Zusammensetzung zur Verwendung nach Anspruch 1, **dadurch gekennzeichnet, dass** sie bei der Behandlung von Unfruchtbarkeit bei Männern, die Asthenospermie und/oder Teratospermie aufweisen, verwendet wird.

3. Zusammensetzung zur Verwendung nach einem der vorstehenden Ansprüche, **dadurch gekennzeichnet, dass** sie ferner Koenzym Q10 umfasst.

4. Zusammensetzung zur Verwendung nach einem der vorstehenden Ansprüche, **dadurch gekennzeichnet, dass** sie ferner mindestens eine Aminosäure, ein Carotinoid, ein Spurenelement, ein Vitamin und/oder einen Wurzelextrakt umfasst, die einzeln oder in einer Mischung eingenommen werden.

5. Zusammensetzung zur Verwendung nach Anspruch 4, **dadurch gekennzeichnet, dass:**
- die Aminosäure Lysin, Methionin oder Carnitin, vorzugsweise L-Carnitin, ist;
- das Carotinoid Lycopin ist;
- das Spurenelement aus Zink und Selen ausgewählt ist;
- das Vitamin aus Vitamin B6, Vitamin B9, Vitamin B12, Vitamin C, Vitamin D und Vitamin E ausgewählt ist; und
- der Wurzelextrakt ein Extrakt aus der Maca-Wurzel (*Lepidium meyenii*) ist.

6. Zusammensetzung zur Verwendung nach einem der Ansprüche 4 und 5, **dadurch gekennzeichnet, dass** sie ein Spurenelement, das Zink ist, umfasst.

7. Zusammensetzung zur Verwendung nach einem der vorstehenden Ansprüche, **dadurch gekennzeichnet, dass** sie auf oralem Weg verabreicht wird.

8. Zusammensetzung zur Verwendung nach Anspruch 7, **dadurch gekennzeichnet, dass** sie in Form einer Tablette, einer Hartkapsel, einer Kapsel, eines weichen Gels, halbfest, fest, einer Flüssigkeit oder eines Pulvers formuliert ist.

9. Zusammensetzung zur Verwendung nach Anspruch 8, **dadurch gekennzeichnet, dass** sie 1-mal täglich verabreicht wird und dass sie in Form einer Tablette, deren Gewicht vorzugsweise 650 mg beträgt, vorliegt.

10. Zusammensetzung zur Verwendung nach einem der vorstehenden Ansprüche, **dadurch gekennzeichnet, dass** sie während eines Zeitraums von mindestens drei Monaten verabreicht wird.

11. Zusammensetzung zur Verwendung nach einem der vorstehenden Ansprüche bei der Behandlung und/oder der Vorbeugung von Unfruchtbarkeit, die nicht mit einer mikrobakteriell bedingten Entzündung wie Prostatitis verbunden ist.

## Claims

1. Composition comprising:
- an aqueous pollen extract from *Secale cereale* L.;
- an aqueous pollen extract from *Zea mays* L.;
- an aqueous pollen extract from *Pinus silvestris* L.;
- an aqueous pollen extract from *Dactylis glomerata* L.; and
- an aqueous pistil extract from *Zea mays* L.,
for use in the treatment and/or prevention of infertility in men with oligospermia, cryptozoospermia, azoospermia, asthenospermia, oligoasthenospermia and/or teratospermia,
**characterized in that** the pollen extract and the pistil extract are aqueous extracts, the extraction of which is carried out at a temperature of less than 45°C.

2. Composition for use according to claim 1, **characterized in that** it is used in the treatment of infertility in men with asthenospermia and/or teratospermia.

3. Composition for use according to one of the preceding claims, **characterized in that** it further comprises coenzyme Q10.

4. Composition for use according to one of the preceding claims, **characterized in that** it further comprises at least one amino acid, a carotenoid, a trace element, a vitamin and/or a root extract, taken alone or in a mixture.

5. Composition for use according to claim 4, **characterized in that:**
- the amino acid is lysine, methionine or carnitine, preferably L-carnitine;
- the carotenoid is lycopene;
- the trace element is selected from zinc and selenium;
- the vitamin is selected from vitamin B6, vitamin B9, vitamin B12, vitamin C, vitamin D and vitamin E; and
- the root extract is maca root extract *(Lepidium meyenii).*

6. Composition for use according to one of claims 4 and 5, **characterized in that** it comprises a trace element which is zinc.

7. Composition for use according to one of the preceding claims, **characterized in that** it is administered orally.

8. Composition for use according to claim 7, **characterized in that** it is formulated as a tablet, gelcap, capsule, soft gel, semi-solid, solid, liquid or powder.

9. Composition for use according to claim 8, **characterized in that** it is administered once daily and **in that** it is in the form of a tablet, the weight of which is preferably 650 mg.

10. Composition for use according to one of the preceding claims, **characterized in that** it is administered over a period of at least three months.

11. Composition for use according to one of the preceding claims, in the treatment and/or prevention of infertility which is not associated with inflammation of microbacterial origin, such as prostatitis.
